# EUROPÄISCHE PATENTSCHRIFT

(11) **EP 1 661 598 B1**
(45) Veröffentlichungstag und Bekanntmachung des Hinweises auf die Patenterteilung: **31.10.2007**
(21) Anmeldenummer: 05020070.8
(22) Anmeldetag: 15.09.2005
(51) Int. Cl.: A61M 25/06

(54) **Piercingnadel**
Piercing needle
Aiguille de perçage

(30) Priorität: 30.11.2004 DE 102004057785
(43) Veröffentlichungstag der Anmeldung: 31.05.2006
(73) Patentinhaber: Raths, Arno, 52080 Aachen (DE)
(72) Erfinder: Raths, Arno, 52080 Aachen (DE)
(74) Vertreter: Bauer, Dirk

(56) Entgegenhaltungen:
- EP-A- 0 613 695
- EP-A- 1 252 834
- DE-A1- 3 915 215
- US-A- 4 994 036
- US-A- 6 027 480

## Beschreibung

Die Erfindung betrifft eine Piercingnadel mit einer Nadel, die an einem Ende eine Spitze aufweist und an ihrem anderen Ende fest mit einem Griffstück verbunden ist. Sie ist in einen Katheter herausziehbar eingeschoben, wobei der Katheter kürzer als die Nadel ist.

Unter Piercing versteht man das Durchstechen von Haut und eventuell darunter liegendem Fett- oder Knorpelgewebe zur Anbringung von Körperschmuck. Beliebte Stellen sind zum Beispiel Ohrläppchen, Nasenwand, Augenbrauen, Lippen, Bauchnabel oder der Intimbereich. Seit den neunziger Jahren erfreut sich das Piercing in größeren Bevölkerungsschichten einer wachsenden Beliebtheit. Besonders bei nicht fachgerecht durchgeführten Piercings kommt es jedoch häufig zu Komplikationen oder Infektionen. Je nach Körperstelle und Pflege kann der Heilungsprozess recht langwierig sein und bis zu einigen Monaten dauern. Die fachgerechte Durchführung von Piercings wird dadurch erschwert, dass bislang kein ausgesprochenes Piercingwerkzeug zur Verfügung steht und statt dessen zweckentfremdete Werkzeuge wie Venenverweilkanüten oder Biopsiehohlnadeln verwendet werden, wobei häufig Probleme auftreten. Ein Katheter Set mit den Merkmalen des Oberbegriffs von Patentanspruch 1 ist beispielsweise aus US-A-4994036 bekannt.

Nach dem derzeitigen Stand der Technik werden zum Piercen meist Venenverweilkanülen verwendet. Venenverweilkanülen bestehen aus einer Kanüle, welche von einem Katheter umhüllt ist. Der Katheter ist fest mit einer Infusionsaufnahme verbunden. Für eine Infusion - beispielsweise in den Handrücken - wird die Kanüle mitsamt dem Katheter in die Vene eingestochen. Die Infusionsaufnahme wird dann an dem Handrücken mit einem Pflaster befestigt und anschließend die Kanüle aus dem Katheter herausgezogen. Der Katheter mit der Infusionsaufnahme bleibt in der Vene stecken, und ein Infusionsschlauch kann an die Infusionsaufnahme angeschlossen werden.

Bei der Verwendung von solchen Venenverweilkanülen für das Piercing sieht der Arbeitsablauf meist folgendermaßen aus:
Zunächst wird die zu piercende Stelle desinfiziert und mit einem sterilen Stift markiert. Mit einer Hand wird dann die entsprechende Stelle mit einer Zange festgehalten und mit der zweiten Hand die Venenverweilkanüle durch die markierte Stelle gestochen. Anschließend wird die Kanüle an einem Griff aus dem Katheter, welcher in der Stichöffnung zurückbleibt, gezogen. Nun wird die Infusionsaufnahme, welche fest mit dem Katheter verbunden ist, mit einer Schere abgeschnitten und ein Schmuckstück in die Schnittöffnung des Katheters eingesetzt. Der Schmuck wird dann zusammen mit dem Katheter durch die Stichöffnung im Gewebe gezogen.

Dabei treten folgende Probleme auf:
Die auf dem Markt befindlichen Venenverweilkanülen sind für die beidhändige Bedienung ausgelegt.
Beim Piercen wird jedoch in der Regel eine Hand für das Halten der Zange benötigt. Die einhändige
Bedienung gestaltet sich als schwierig, da die mit dem Katheter verbundene Infusionsaufnahme fest auf dem Griff der Kanüle sitzt und zur Trennung ein gewisser Kraftaufwand erforderlich ist. Dies wird zusätzlich dadurch erschwert, dass die Infusionsaufnahme, welche für einen völlig anderen Zweck konzipiert wurde, dabei keine ausreichende Griffsicherheit bietet und man bei einhändiger Bedienung leicht abrutschen kann.

Für Piercings an bestimmten Körperstellen muss die Kanüle zuerst in eine Körperöffnung eingeführt werden, bevor tatsächlich Gewebe durchstochen wird. Dies ist beispielsweise bei dem so genannten "Prinz Albert"-Piercing der Fall. Dabei wird die Kanüle zunächst in die Harnröhre geschoben, um dann am Vorhautband von innen durch das Gewebe gestochen zu werden. Für derartige Anwendungen ist es wichtig, dass der Katheter zunächst schützend über die Kanülenspitze hinaus nach vorne geschoben wird, um Verletzungen zu vermeiden. Erst wenn die Kanüle genügend tief eingeschoben und der Punkt erreicht wurde, an dem Gewebe durchstochen werden soll, wird dann der Katheter wieder auf der Kanüle zurückgeschoben, so dass die Spitze frei ist. Durch die mangelnde Griffsicherheit an der Infusionsaufnahme ist es dabei oft schwierig, einen einmal über die Kanülenspitze hinausgeschobenen Katheter wieder in die Ausgangsposition zurückzuschieben. Einige Venenverweilkanülen haben sogar Arretierungen, die dies verhindern sollen. Dies hat zur Folge, dass der Katheter oft ungenügend weit zurückgeschoben wird und er zumindest teilweise noch über der Kanülenspitze steckt. Beim Durchstechen von Gewebe kann dieser dann leicht splittern oder reißen, was ein enormes Verletzungsrisiko mit sich bringt.

Ein weiteres Problem bei der Verwendung von Venenverweilkanülen besteht darin, dass die Infusionsaufnahme fest mit dem Katheter verbunden ist. Um einen Schmuck einzusetzen und mit dem Katheter durch die Stichöffnung im Gewebe zu ziehen, muss die Infusionsaufnahme zuvor abgetrennt werden. Dies geschieht häufig mit einer Schere. Dabei lässt es sich in der Regel nicht vermeiden, dass der Katheter im Querschnitt gequetscht wird oder der Katheter ausfranst und dass scharfe Kanten entstehen. Dies erschwert einerseits das Einsetzen des Schmuckes und birgt vor allem eine erhöhte Verletzungsgefahr, wenn der scharfkantige Katheter durch die Stichöffnung im Gewebe gezogen wird.

Vor diesem Hintergrund ist es Aufgabe der Erfindung, eine Piercingnadel anzugeben, welche unter Vermeidung der oben beschriebenen Probleme speziell für diesen Zweck konzipiert ist. Insbesondere soll eine Piercingnadel angegeben werden, welche mit einer Hand sicher zu bedienen ist und welche das Verletzungsrisiko gegenüber dem derzeitigen Stand der Technik reduziert.

Die Aufgabe wird durch die Erfindung gemäß dem unabhängigen Anspruch 1 gelöst. Bevorzugte Weiterbildungen sind den Unteransprüchen zu entnehmen.

Eine erfindungsgemäße Piercingnadel umfasst eine Nadel, die an einem Ende eine Spitze aufweist und an ihrem anderen Ende fest mit einem ersten Griffstück verbunden ist. Die Nadel ist in einen Katheter herausziehbar eingeschoben, wobei der Katheter kürzer ist als die Nadel. Die erfindungsgemäße Piercingnadel weist ein Zusatzgriffstück auf, das derart verschiebbar zwischen dem Katheter und dem ersten Griffstück auf der Nadel sitzt, dass durch Verschieben des Zusatzgriffstücks in Richtung der Nadelspitze der Katheter in die gleiche Richtung verschoben werden kann.

Die Spitze der Nadel kann verschiedenartig ausgeformt sein. Ein Schliff ist jedoch bevorzugt, da das Einstechen in Gewebe dann mit einem Schnitt verbunden ist und dadurch weniger Schmerzen verursacht. An ihrem anderen Ende ist die Nadel fest in einem Griffstück verankert. Dieses dient einerseits der sicheren Führung der Nadel beim Einstechen, andererseits wird daran die Nadel nach dem Durchstechen des Gewebes herausgezogen. Die Nadel ist in an sich bekannter Weise herausziehbar in einen Katheter eingeschoben. Katheter und Nadel sind gegeneinander verschiebbar, und die Nadel kann aus dem Katheter völlig herausgezogen werden. Zwischen dem Katheter und dem ersten Griffstück steckt ein Zusatzgriffstück verschiebbar auf der Nadel, so dass der Katheter mit Hilfe des Zusatzgriffstücks auf der Nadel in Richtung zur Nadelspitze hin geschoben werden kann. Katheter und Zusatzgriffstück sind dabei nicht fest miteinander verbunden. Der Innendurchmesser des Katheters ist im Allgemeinen nur wenig größer als der Außendurchmesser der Nadel, so dass der Katheter wie eine Schutzhülle an der Nadel anliegt, ohne jedoch die Verschiebbarkeit einzuschränken. Der Katheter ist etwas kürzer als die Nadel und reicht im zurückgeschobenen Zustand, das heißt, wenn der Katheter an das Zusatzgriffstück anschlägt und dieses seinerseits an das erste Griffstück anschlägt, bis zur Spitze der Nadel, ohne diese zu bedecken. In diesem eingeschobenen Zustand ragt die Nadel also nur mit ihrer Spitze aus dem Katheter heraus.

Die Anwendung der Piercingnadel erfolgt im Prinzip ähnlich wie bei den bisher verwendeten Venenverweilkanülen, jedoch ist die Griffsicherheit durch das Zusatzgriffstück extrem vereinfacht und ein Durchtrennen des Katheters, wie zum Abschneiden der Infusionsaufnahme bei einer Venenverweilkanüle, ist nicht mehr nötig, da Katheter und Zusatzgriffstück nicht fest miteinander verbunden sind.

In einer Ausführungsvariante sitzt der Katheter lediglich lose in dem Zusatzgriffstück, so dass der Katheter beim Vorschieben des Zusatzgriffstücks in Richtung zur Nadelspitze mit nach vorne geschoben wird, während er beim Zurückziehen des Zusatzgriffstücks in die entgegengesetzte Richtung in vorgeschobener Stellung zurückbleibt. Wenn diese Ausführungsvariante in das Gewebe eingestochen und die Nadel anschließend zurückgezogen wird, bleibt der Katheter automatisch in der Stichöffnung zurück und das Zusatzgriffstück fällt ab.

Bei einer anderen Ausführungsvariante steckt der Katheter im Klemmsitz in dem Zusatzgriffstück, so dass der Katheter mit Hilfe des Zusatzgriffstücks auf der Nadel vor- und zurück geschoben werden kann. Diese Ausführungsvariante ist besonders geeignet für das oben beschriebene "Prinz Albert"-Piercing. Vor dem Einschieben der Nadel in die Harnröhre wird der Katheter mit Hilfe des Zusatzgriffstücks schützend über die Nadelspitze geschoben, um Verletzungen der Harnröhre zu vermeiden. Vor dem Durchstechen des Gewebes wird der Katheter dann wieder mit Hilfe des Zusatzgriffstücks in seine Ausgangsposition zurückgeschoben, so dass die Spitze der Nadel ungehindert durch das Gewebe stechen kann. Der Klemmsitz zwischen Zusatzgriffstück und Katheter darf dabei jedoch nur so stark sein, dass sich Katheter und Zusatzgriffstück nach dem Herausziehen der Nadel leicht mit einer Hand trennen lassen. Hier muss nur gewährleistet sein, dass die Haftung zwischen Zusatzgriffstück und Katheter stärker ist als zwischen Katheter und Nadel.

In einer bevorzugten Weiterbildung ist die Nadel, zumindest in einem ihrer Spitze abgewandten Teil, mit einer Gleitschicht versehen. Diese Gleitschicht vermindert die Reibung zwischen Katheterinnenwand und Nadel. Die Spitze der Nadel ist von der Gleitschicht freigehalten, um ungehindert in das Gewebe einstechen zu können. In einer besonders bevorzugten Variante besteht diese Gleitschicht aus einem Kunststoff mit einem niedrigen Gleitreibungskoeffizienten. Dies vereinfacht besonders bei längeren Nadeln, wie sie für das "Prinz Albert"-Piercing verwendet werden, das Verschieben des Katheters auf der Nadel.

Der Begriff Nadel umfasst hier sowohl kompakte Nadeln als auch Hohlnadeln wie zum Beispiel Kanülen. Vorzugsweise ist die Nadel eine Kanüle, wobei die Spitze durch einen Schliff gebildet sein kann.

Bevorzugterweise ist der Katheter zur Nadelspitze hin verjüngt. Dies erleichtert das Einstechen in Gewebe.

Die Piercingnadel sollte zur Sicherheit mit einem Transportschutz ausgestattet sein, der zur Verwendung abgenommen wird. Der Transportschutz sollte dabei so ausgestaltet sein, dass er einerseits Nadel und Katheter vor Beschädigungen schützt und andererseits einen sicheren Verletzungsschutz für handhabende Personen bietet. Eine Möglichkeit ist ein einfaches Rohr, beispielsweise aus stabilem Kunststoff, das über Nadel und Katheter gesteckt wird, diese in ihrer Länge überragt und im Klemmsitz auf einem der Griffstücke befestigt wird. Der Transportschutz kann dabei offen oder geschlossen sein.

In einer bevorzugten Weiterbildung hat das Zusatzgriffstück der Piercingnadel mindestens eine Griffmulde und/oder mindestens einen Griffvorsprung. Dies erleichtert die Handhabung insbesondere mit einer Hand. Bei der einhändigen Bedienung liegt das erste Griffstück der Piercingnadel beispielsweise so in der Hand, dass es zwischen Handballen und Mittel-, Ring- und kleinem Finger eingeklemmt wird, während das Zusatzgriffstück mit Daumen und Zeigefinger bewegt wird - ähnlich wie beim einhändigen Öffnen einer Stiftkappe. Griffmulde oder Griffvorsprung sollen dabei ein Abrutschen von Daumen und Zeigefinger an dem Zusatzgriffstück verhindern. Dabei hat das Zusatzgriffstück vorzugsweise eine Länge zwischen 0,5 und 4 cm. Griffmulde und/oder Griffvorsprung sollen sowohl das Vorals auch das Zurückschieben des Zusatzgriffstücks auf der Nadel erleichtern.

Für einen sicheren Halt der Piercingnadel in einer Hand sollte auch das erste Griffstück eine ausreichende Länge aufweisen. Diese kann zwischen 2 und 8 cm betragen. Zweckmäßig wird die Länge so gewählt, dass das erste Griffstück sicher in der Hand abgestützt werden kann, während das Zusatzgriffstück mit zwei Fingern vor- und zurückbewegt wird.

Nadel und Zusatzgriffstück sollten dabei so abgemessen sein, dass die Nadel um eine ausreichende Länge aus dem Zusatzgriffstück hervorsteht, um eine Gewebeschicht von der gewünschten Dicke durchstoßen zu können. Diese Nadellänge beträgt vorzugsweise zwischen 3 cm und 10 cm.

In einer bevorzugten Weiterbildung ist der Innendurchmesser des Katheters der Größe des Schmuckstückes angepasst. Maßgeblich ist dabei zumindest der Teil des Schmuckes, der durch die Stichöffnung geführt werden soll. Das Schmuckstück kann aus einem oder mehreren Teilen bestehen. Der Begriff Schmuckstück umfasst hier alle zugehörigen Teile, also auch eventuell vorhandene Befestigungsteile. Vorzugsweise ist der Innendurchmesser des Katheters um etwa 0,1 mm kleiner als der Durchmesser des betreffenden Schmuckteils. Das Schmuckstück kann dann in das offene Katheterende eingesteckt werden. Durch den relativ engen Katheterdurchmesser wird der Schmuck eingeklemmt und so festgehalten. Auf diese Weise kann dann das Schmuckstück mit dem Katheter durch die Stichöffnung im Gewebe gezogen werden.

Als Materialien für die erfindungsgemäße Piercingnadel können die aus dem Stand der Technik bekannten Materialien für herkömmliche Piercingnadeln oder Venenverweilkanülen verwendet werden. Die Nadel kann beispielsweise aus Metall oder Keramik hergestellt sein, vorzugsweise besteht sie aus Metall. Der Katheter, das erste Griffstück und das Zusatzgriffstück können dagegen aus Kunststoff oder aus einem anderen geeigneten Material gefertigt sein, das nicht unbedingt gegen Sterilisation oder hohe Temperaturen beständig sein muss. Dies soll verhindern, dass die Piercingnadel in einem Heißluft- oder Dampfsterilisator erneut aufbereitet und wiederverwendet werden kann. Vorzugsweise ist die Piercingnadel steril und einzeln steril verpackt und damit als Einweg-Werkzeug verwendbar. Andererseits ist es auch denkbar, die Piercingnadel aus solchen Materialien zu fertigen, die sich mit konventionellen Methoden sterilisieren lassen. Eine wiederholte Verwendung wäre dann denkbar. Aus Sicherheits- und Hygienegründen sind sterile Einweg-Piercingnadeln jedoch zu bevorzugen.

Nachfolgend wird die Erfindung anhand von zwei in den Figuren dargestellten Ausführungsbeispielen weiter erläutert. Es zeigen schematisch:
- Fig. 1: eine erste Ausführungsvariante einer erfindungsgemäßen Piercingnadel im Längsschnitt;
- Fig. 2: die gleiche Ausführungsvariante einer Piercingnadel im Längsschnitt ohne Transportschutz mit auseinandergeschobenen Komponenten;
- Fig. 3: die Spitze einer Nadel mit sich verjüngendem Ende eines Katheters;
- Fig. 4: eine zweite Ausführungsvariante im Längsschnitt;
- Fig. 5: die zweite Ausführungsvariante ohne Transportschutz mit auseinandergeschobenen Komponenten;
- Fig. 6: bis Fig. 10 den Verfahrensablauf beim Piercing mit einer Piercingnadel der ersten Ausführungsvariante, wie in Fig. 1 dargestellt. Dabei zeigen im Einzelnen: Fig. 6 zeigt die Piercingnadel vor dem Einstechen in Gewebe;
- Fig. 7: die Piercingnadel beim Herausziehen der Nadel aus dem Katheter;
- Fig. 8: die Piercingnadel, nachdem die Nadel aus dem Katheter herausgezogen und das Zusatzgriffstück von dem Katheter gelöst wurde;

- Fig. 9: das Einsetzen eines Schmuckstückes in den Katheter;
- Fig. 10: das Durchziehen des Katheters mit dem Schmuckstück durch den Stichkanal im Gewebe und
- Fig. 11: das Schmuckstück im eingesetzten Zustand.

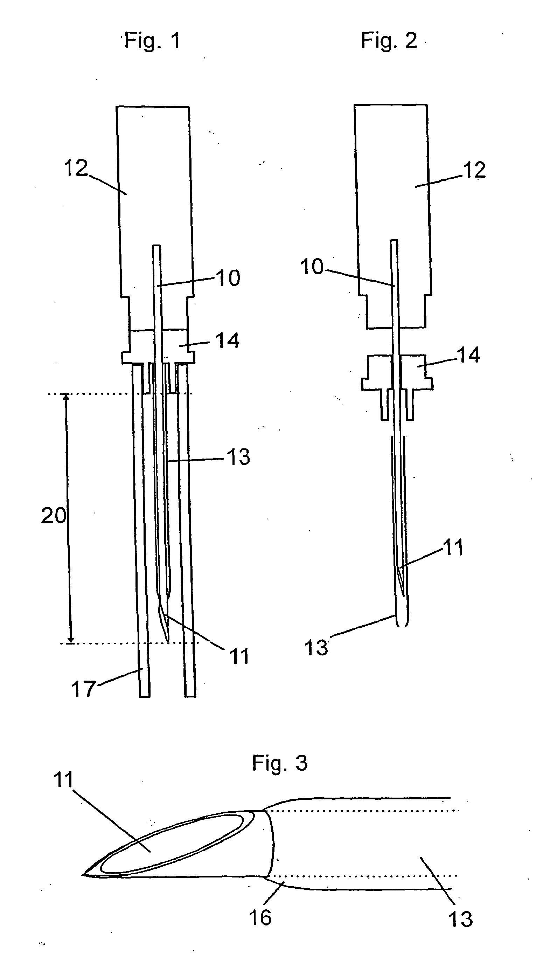

Die Piercingnadel in Fig. 1 besteht aus einer Nadel 10, hier in Form einer Kanüle mit einem Schliff an der Spitze 11, die an ihrem anderen Ende fest mit einem im Wesentlichen zylindrischen Griffstück 12 verbunden ist. Die Kanüle ist eingeschoben in einen Katheter 13, der sich zur Kanülenspitze hin verjüngt und der an seinem anderen Ende lose an einem Zusatzgriffstück 14 ansteht. Das Zusatzgriffstück 14, welches zwischen Katheter und erstem Griffstück 12 auf der Kanüle steckt, hat einen Griffvorsprung 19, um einen sicheren Halt zu gewährleisten. Zum Schutz der Kanüle und zum Schutz vor Verletzungen durch die Kanüle ist die Piercingnadel mit einem Transportschutz 17 ausgestattet. Der Transportschutz ist im Klemmsitz an dem Zusatzgriffstück 14 befestigt und in Richtung zur Nadelspitze hin abziehbar.

Dieselbe Piercingnadel ist in Fig. 2 mit auseinandergeschobenen Komponenten ohne Transportschutz dargestellt. Sowohl Katheter 13 als auch Zusatzgriffstück 14 sind auf der Nadel 10 verschiebbar, wobei der Katheter lose in dem Zusatzgriffstück sitzt und mit diesem in Richtung zur Nadelspitze 11 geschoben werden kann. Zieht man dagegen das vorgeschobene Zusatzgriffstück 14 zurück in Richtung des ersten Griffstücks 12, so folgt der Katheter 13 nicht, sondern Zusatzgriffstück und Katheter lösen sich voneinander.

Damit der Katheter 13 beim Einstechen in Gewebe leicht in die Stichöffnung eindringen kann, ist er zu seinem der Nadelspitze 11 zugewandten Ende 16 hin verjüngt. Fig. 3 zeigt die Spitze einer Kanüle mit Schliff, die in einen sich zur Spitze verjüngenden Katheter 13 eingeschoben ist.

Eine solche Piercingnadel eignet sich für Piercings an leicht zugänglichen Körperpartien wie Ohrläppchen, Augenbrauen, Lippen oder Bauchnabel. Die Nadellänge 20 vor dem Zusatzgriffstück kann beispielsweise etwa 50 mm betragen.

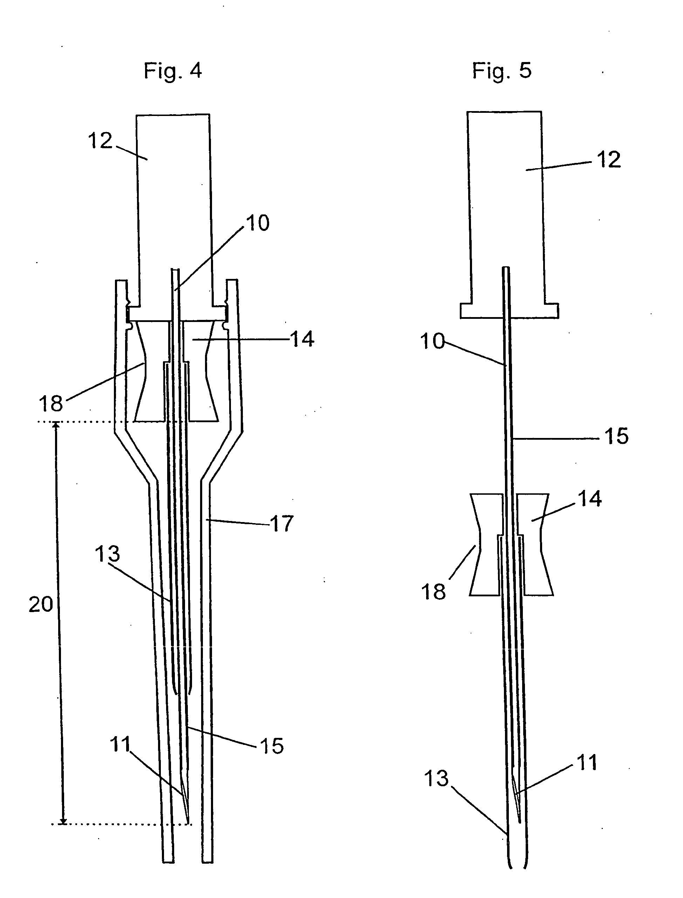

In den Fig. 4 und Fig. 5 ist eine andere Ausführungsvariante einer erfindungsgemäßen Piercingnadel dargestellt. Dieses Modell ist für Piercings gedacht, bei denen die Nadel vor dem eigentlichen Stich in eine Körperöffnung eingeführt wird, wie zum Beispiel in die Harnröhre für ein "Prinz Albert"-Piercing. Die Länge 20 der Nadel ist hier dementsprechend größer und kann etwa 80 mm betragen. Damit sich der eng anliegende Katheter 13 und die Nadel 10 trotz der großen Länge 20 noch leicht gegeneinander verschieben lassen, ist die Nadel 10 dieser Ausführung mit einer Gleitschicht 15 (dargestellt durch eine verstärkte Umfangslinie) versehen. Der Katheter steckt im Klemmsitz in einem Zusatzgriffstück 14, so dass er mit diesem zusammen entlang der Nadel 10 vor- und zurückgeschoben werden kann. Dadurch kann der Katheter 13 vor dem Einführen in die Harnröhre mit dem Zusatzgriffstück 14 über die Nadelspitze 11 hinausgeschoben werden. Derart geschützt wird dann die Nadel in die Harnröhre eingeführt, bis der Punkt zum Durchstechen des Gewebes erreicht ist. An dieser Stelle wird dann der Katheter 13 mit Hilfe des Zusatzgriffstückes 14 auf der Nadel 10 zurückgezogen. Oder die Nadel 10 wird nachgeschoben, so dass die Spitze 11 der Nadel freigelegt ist und ungehindert in das Gewebe einstechen kann. Das Zusatzgriffstück ist zu diesem Zweck mit einer symmetrischen Griffmulde 18 ausgestattet, die einen sicheren Halt beim Verschieben in beide Richtungen erlaubt. Fig. 4 zeigt die Piercingnadel im zusammengeschobenen Zustand mit Transportschutz 17, der an dem ersten Griffstück 12 festgemacht ist. In Fig. 5 ist dieselbe Piercingnadel mit vorgeschobenem Katheter 13 ohne Transportschutz 17 zu sehen.

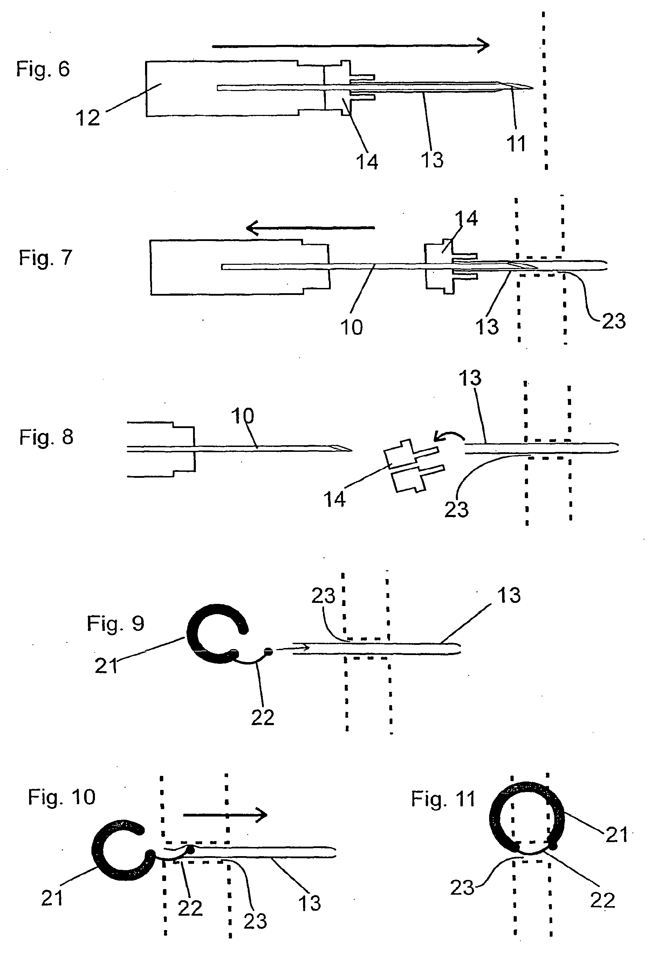

Fig. 6 bis Fig. 10 zeigen schrittweise die Anwendung einer Piercingnadel der ersten Art, wie sie in den Figuren 1 und 2 dargestellt ist.

Fig. 6 zeigt die Piercingnadel im Grundzustand, bevor sie in Gewebe (angedeutet durch die gestrichelte Linie) eingestochen wird. Die Bewegungsrichtung ist durch den Pfeil angezeigt. Bei diesem ersten Schritt sind Katheter 13 und Zusatzgriffstück 14 bis zum Anschlag an das erste Griffstück 12 zurückgeschoben. Nadel 10 und Katheter 13 werden dann gemeinsam soweit durch das Gewebe gestochen, dass sie an beiden Seiten aus dem entstandenen Stichkanal herausragen. Im zweiten Schritt (Fig. 7) wird die Nadel 10 am ersten Griffstück 12 aus dem Stichkanal 23 gezogen. Der Katheter 13 bleibt dabei im Stichkanal 23 stecken. Ein unbeabsichtigtes Herausziehen des Katheters kann mit Hilfe des Zusatzgriffstückes 14 verhindert werden. Nachdem die Nadel ganz herausgezogen wurde, lässt sich im dritten Schritt (Fig. 8) auch das Zusatzgriffstück leicht von dem Katheter lösen. Ein Abschneiden des Zusatzgriffstückes vom Katheter ist nicht nötig. Daher können scharfe Schnittkanten und Quetschungen des Katheters vermieden werden. Nun wird im vierten Schritt (Fig. 9) ein Schmuckstück 21, beispielsweise ein Ring, eingesetzt. Dabei wird der Teil 22 des Schmuckes, der durch den Stichkanal 23 ragen soll, in den Katheter 14 eingeführt, darin verklemmt und anschließend mit dem Katheter durch den Stichkanal gezogen (Fig. 10). Zuletzt kann der Schmuck 21 auf der Rückseite fixiert werden (Fig. 11).

Durch die vorgestellte Piercingnadel kann das Risiko von Verletzungen oder nachfolgenden Komplikationen durch das Piercing stark herabgesetzt werden.

## Patentansprüche

1. Piercingnadel mit einem Katheter (13) und mit einer Nadel (10), die an einem Ende eine Spitze (11) aufweist, an ihrem anderen Ende fest mit einem ersten Griffstück (12) verbunden, und die in dem Katheter (13) herausziehbar eingeschoben ist, wobei der Katheter (13) kürzer als die Nadel (10) ist und wobei die Nadel (10) ein Zusatzgriffstück (14) aufweist, das derart verschiebbar zwischen dem Katheter (13) und dem ersten Griffstück (12) auf der Nadel (10) sitzt, dass durch Verschieben des Zusatzgriffstücks (14) in Richtung der Spitze (11) der Nadel (10) der Katheter (13) in die gleiche Richtung verschoben wird,
**dadurch gekennzeichnet, dass**
der Katheter (13) lose in dem Zusatzgriffstück (14) sitzt.

2. Piercingnadel nach Anspruch 1,
**dadurch gekennzeichnet, dass** der Katheter (13) im Klemmsitz in dem Zusatzgriffstück (14) steckt, so dass der Katheter (13) mit Hilfe des Zusatzgriffstücks (14) auf der Nadel (10) vor- und zurückgeschoben werden kann.

3. Piercingnadel nach Anspruch 1 oder 2,
**dadurch gekennzeichnet, dass** die Nadel (10) zumindest in einem von ihrer Spitze (11) abgewandten Teil mit einer Gleitschicht (15) versehen ist.

4. Piercingnadel nach Anspruch 3,
**dadurch gekennzeichnet, dass** die Gleitschicht (15) aus einem Kunststoff mit niedrigem Gleitreibungskoeffizienten besteht.

5. Piercingnadel nach einem der vorigen Ansprüche,
**dadurch gekennzeichnet, dass** die Nadel (10) eine Kanüle ist.

6. Piercingnadel nach einem der vorigen Ansprüche,
**dadurch gekennzeichnet, dass** sich der Katheter (13) zur Nadelspitze (11) hin verjüngt (16).

7. Piercingnadel nach einem der vorigen Ansprüche,
**dadurch gekennzeichnet, dass** sie einen abnehmbaren Transportschutz (17) zum Schutz von Nadel (10) und Katheter (13) aufweist.

8. Piercingnadel nach einem der vorigen Ansprüche,
**dadurch gekennzeichnet, dass** der Transportschutz (17) im Klemmsitz so auf einem der Griffstücke (12, 14) sitzt, dass er die Nadel (10) und den Katheter (13) sicher umschließt.

9. Piercingnadel nach einem der vorigen Ansprüche,
**dadurch gekennzeichnet, dass** das Zusatzgriffstück (14) mindestens eine Griffmulde (18) und/oder mindestens einen Griffvorsprung (19) hat.

10. Piercingnadel nach einem der vorigen Ansprüche,
**dadurch gekennzeichnet, dass** das Zusatzgriffstück (14) eine Länge zwischen 0,5 und 4 cm besitzt.

11. Piercingnadel nach einem der vorigen Ansprüche,
**dadurch gekennzeichnet, dass** das erste Griffstück (12) eine Länge in der Größenordnung von 2 bis 8 cm aufweist.

12. Piercingnadel nach einem der vorigen Ansprüche,
**dadurch gekennzeichnet, dass** im zusammengeschobenen Zustand die Nadel (10) um eine Länge (20) zwischen 3 cm und 10 cm über das Zusatzgriffstück (14) hinausragt.

13. Piercingnadel nach einem der vorigen Ansprüche,
**dadurch gekennzeichnet, dass** der Innendurchmesser des Katheters (13) dem Außendurchmesser eines einzusetzenden Schmuckes (21) zumindest in dem Teil des Schmuckes (22), der zum Durchziehen durch die durch das Piercing entstandene Gewebeöffnung (23) in den Katheter gesteckt werden soll, angepasst ist.

14. Piercingnadel nach einem der vorigen Ansprüche,
**dadurch gekennzeichnet, dass** dass die Nadel (10) aus Metall besteht.

15. Piercingnadel nach einem der vorigen Ansprüche,
**dadurch gekennzeichnet, dass** der Katheter (13), das erste Griffstück (12) und das Zusatzgriffstück (14) aus Kunststoff gefertigt sind.

## Claims

1. Piercing needle comprising a catheter (13) and comprising a needle (10) which at one end has a tip (11), at its other end is rigidly connected to a first handle (12) and is extractably inserted into the catheter (13), the catheter (13) being shorter than the needle (10) and the needle (10) having an additional handle (14) which rests on the needle (10) so as to be displaceable between the catheter (13) and the first handle (12) in such a way that displacing the additional handle (14) toward the tip (11) of the needle (10) causes the catheter (13) to be displaced in the same direction, **characterised in that** the catheter (13) rests loosely in the additional handle (14).

2. Piercing needle according to claim 1, **characterised in that** the catheter (13) is press-fitted in the additional handle (14), so the catheter (13) can be pushed back and forth on the needle (10) using the additional handle (14).

3. Piercing needle according to either claim 1 or claim 2, **characterised in that** the needle (10) is provided with a sliding layer (15), at least in a portion remote from its tip (11).

4. Piercing needle according to claim 3, **characterised in that** the sliding layer (15) is made of a plastics material having a low coefficient of sliding friction.

5. Piercing needle according to any one of the preceding claims, **characterised in that** the needle (10) is a cannula.

6. Piercing needle according to any one of the preceding claims, **characterised in that** the catheter (13) tapers (16) toward the tip (11) of the needle.

7. Piercing needle according to any one of the preceding claims, **characterised in that** it has a removable transport protector (17) for protecting the needle (10) and catheter (13).

8. Piercing needle according to any one of the preceding claims, **characterised in that** the transport protector (17) is press-fitted to one of the handles (12, 14) so as securely to surround the needle (10) and the catheter (13).

9. Piercing needle according to any one of the preceding claims, **characterised in that** the additional handle (14) has at least one recessed grip (18) and/or at least one gripping projection (19).

10. Piercing needle according to any one of the preceding claims, **characterised in that** the additional handle (14) has a length of between 0.5 and 4 cm.

11. Piercing needle according to any one of the preceding claims, **characterised in that** the first handle (12) has a length of approximately from 2 to 8 cm.

12. Piercing needle according to any one of the preceding claims, **characterised in that** the needle (10), when assembled, protrudes beyond the additional handle (14) by a length (20) of between 3 cm and 10 cm.

13. Piercing needle according to any one of the preceding claims, **characterised in that** the internal diameter of the catheter (13) is adapted to the external diameter of an item of jewellery (21) to be inserted, at least in the portion of the item of jewellery (22) that is to be inserted into the catheter for pulling through the opening (23) in the tissue produced by the piercing.

14. Piercing needle according to any one of the preceding claims, **characterised in that** the needle (10) is made of metal.

15. Piercing needle according to any one of the preceding claims, **characterised in that** the catheter (13), the first handle (12) and the additional handle (14) are made of plastics material.

## Revendications

1. Aiguille de perçage (piercing) (10) avec un cathéter (13) et une aiguille (10) qui présente à une extrémité une pointe (11) et est reliée par l'autre extrémité à une poignée (12) et qui peut être insérée de manière extractible dans le cathéter (13), le cathéter (13) étant plus court que l'aiguille (10), et l'aiguille (10) présentant une poignée supplémentaire (14) qui repose sur l'aiguille (10) de manière coulissante entre le cathéter (13) et la première poignée (12) de sorte que par déplacement de la poignée supplémentaire (14) en direction de la pointe (11) de l'aiguille (10), le cathéter (13) est déplacé dans la même direction,
**caractérisée en ce que**
le cathéter (13) repose de manière détachée dans la poignée supplémentaire (14)

2. Aiguille de perçage selon la revendication 1, **caractérisée en ce que** le cathéter (13) est placé en ajustement serré dans la poignée supplémentaire (14) de sorte que le cathéter (13) puisse être avancé et reculé sur l'aiguille (10) à l'aide de la poignée supplémentaire (14).

3. Aiguille de perçage selon la revendication 1 ou 2, **caractérisée en ce que** l'aiguille (10) est munie au moins dans une partie détournée de sa pointe (11) d'une couche antifriction (15).

4. Aiguille de perçage selon la revendication 3, **caractérisé en ce que** la couche antifriction (15) se compose d'une matière plastique avec un coefficient faible de frottement de friction.

5. Aiguille de perçage selon l'une des revendications précédentes, **caractérisé en ce que** l'aiguille (10) est une canule.

6. Aiguille de perçage selon l'une des revendications précédentes, **caractérisée en ce que** le cathéter (13) est rétrécie coniquement (16) en direction de la pointe (11).

7. Aiguille de perçage selon l'une des revendications précédentes, **caractérisée en ce qu'**elle présente une protection de transport amovible (17) pour la protection de l'aiguille (10) et du cathéter (13).

8. Aiguille de perçage selon l'une des revendications précédentes, **caractérisée en ce que** la protection de transport (17) repose en ajustement serré sur l'une des poignées (12,14) de sorte qu'elle entoure de manière sûre l'aiguille (10) et le cathéter (13).

9. Aiguille de perçage selon l'une des revendications précédentes, **caractérisée en ce que** la poignée supplémentaire (14) présente au moins une cavité de préhension (18) et/ou au moins une saillie de préhension (19).

10. Aiguille de perçage selon l'une des revendications précédentes, **caractérisée en ce que** la poignée supplémentaire (14) possède une longueur entre 0,5 et 4 cm.

11. Aiguille de perçage selon l'une des revendications précédentes, **caractérisée en ce que** la première poignée (12) présente une longueur comprise entre 2 et 8 cm.

12. Aiguille de perçage selon l'une des revendications précédentes, **caractérisée en ce qu'**à l'état inséré, l'aiguille (10) dépasse d' une longueur (20) comprise entre 3 cm et 10 cm au-dessus de la poignée supplémentaire (14)

13. Aiguille de perçage selon l'une des revendications précédentes, **caractérisée en ce que** le diamètre interne du cathéter (13) est adapté au diamètre externe d'un bijou à insérer (21) au moins dans la partie de bijou (22), qui doit être insérée dans le cathéter en traversant l'incision de tissu (23) par le perçage.

14. Aiguille de perçage selon l'une des revendications précédentes, **caractérisée en ce que** l'aiguille (10) se compose de métal.

15. Aiguille de perçage selon l'une des revendications précédentes, **caractérisé en ce que** le cathéter (13), la première poignée (12) et la poignée supplémentaire (14) sont fabriqués à partir de matière plastique.
